# EUROPÄISCHE PATENTSCHRIFT

(11) **EP 1 427 731 B1**
(45) Veröffentlichungstag und Bekanntmachung des Hinweises auf die Patenterteilung: **10.05.2006**
(21) Anmeldenummer: 02777064.3
(22) Anmeldetag: 11.09.2002
(51) Int. Cl.: C07D 487/08, C07D 241/00

(54) **VERFAHREN ZUR HERSTELLUNG VON HOCHREINEM TRIETHYLENDIAMIN**
PROCESS FOR THE PREPARATION OF HIGHLY PURE TRIETHYLENE DIAMINE
PROCEDE DE PREPARATION DE TRIETHYLENEDIAMINE TRES PURE

(30) Priorität: 13.09.2001 DE 10145117
(43) Veröffentlichungstag der Anmeldung: 16.06.2004
(73) Patentinhaber: BASF Aktiengesellschaft, 67056 Ludwigshafen (DE)
(72) Erfinder: LANG, Ortmund, 66909 Quirnbach (DE); RUMPF, Bernd, 68766 Hockenheim (DE); FRAUENKRON, Matthias, 67251 Freinsheim (DE); MANDERBACH, Thomas, 67063 Ludwigshafen (DE); STEIN, Bernd, 64665 Alsbach-Hähnlein (DE)
(74) Vertreter: Isenbruck, Günter
(86) Internationale Anmeldenummer: PCT/EP2002/010197
(87) Internationale Veröffentlichungsnummer: WO 2003/022851

(56) Entgegenhaltungen:
- DE-A- 19 962 455

## Beschreibung

Die vorliegende Erfindung betrifft ein Verfahren zur Herstellung von hochreinem Triethylendiamin (TEDA). Dabei wird TEDA verdampft, dieses in dampfförmige. Form in ein flüssiges Lösungsmittel eingeleitet und das TEDA aus der entstandenen Lösung gegebenenfalls isoliert, nachdem vor dem Einleiten des TEDA in ein Lösungsmittel die Schwersieder abgetrennt wurden. Die TEDA-Lösung kann jedoch als solche in den üblichen Anmeldungen ohne weitere Reinigung eingesetzt werden.

TEDA ist ein bedeutender Katalysator für die Herstellung von Polyurethanschäumen. TEDA liegt bei Raumtemperatur als Feststoff vor. Zu seiner Herstellung und auch Reinigung sind verschiedene Verfahren bekannt, u.a. diejenigen, die in den nachfolgend genannten Druckschriften offenbart sind:

DT-A 24 42 929; US 3,297,701; DE-A 36 34 258; DE-A 17 45 627; DE-A 37 18 395; EP-A 111 928; EP-A 382 055; EP-A 842 935, EP-A 842 936; EP-A 831 096; EP-A 952 152 und US 5,741,906.

Die bisher bekannten Verfahren der Herstellung von TEDA führen zu Bildung von Produktgemischen, die neben TEDA noch Wasser, Nebenprodukte wie Piperazin und hochmolekulare Verbindungen sowie das gegebenenfalls bei der Umsetzung eingesetzte Lösungsmittel enthalten. TEDA wird aus diesen Gemischen gewöhnlich durch diskontinuierliche oder kontinuierliche Destillation oder Rektifikation abgetrennt und meist in einem anschließenden Schritt durch Kristallisieren oder Umkristallisieren gereinigt.

TEDA kann ohne Eintreten von Qualitätsverschlechterung, insbesondere der Farbe und Farbstabilität, des Geruchs und der Reinheit, lediglich unter vergleichsweise hohem Aufwand gehandhabt werden.

Für die bekannten, üblichen Anwendungen wird generell ein sehr reines, geruchsloses und reinweißes TEDA gefordert. Die nachfolgend genannten Anmeldungen offenbaren Verfahren, die eine entsprechende TEDA-Qualität liefern sollen:

DT-A 26 11 069; DE-A 28 49 993 und JP-A 49 048 609.

Nachteilig an diesen Verfahren ist, dass sie das TEDA nicht in der gewünschten Qualität liefern.

Die Patentanmeldungen DE 199 33 850.7 vom 23.07.1999 und DE 199 62 455.0 vom 22.12.1999 der Anmelderin betreffen ein Verfahren zur Herstellung von reinem TEDA, bei denen man TEDA verdampft und das dampfförmige TEDA in ein flüssiges Lösungsmittel einleitet sowie das TEDA aus dieser Lösung auskristallisiert.

Die Anmeldung DE 101 00 943.7 der Anmelderin vom 10.01.2001 beschreibt ein Verfahren zur Herstellung einer Lösung von reinem TEDA, das dadurch gekennzeichnet ist, dass man TEDA aus einer Mischung enthaltend ein Lösungsmittel oder ein Verdünnungsmittel, das bei Normaldruck einen Siedepunkt im Bereich von 175 bis 250°C aufweist, verdampft und das dampfförmige TEDA in ein flüssiges Lösungsmittel einleitet. Durch anschließendes Auskristallisieren des TEDA aus der so erhaltenen Lösung wird reines TEDA hoher Qualität erhalten.

Mit den Verfahren nach den beiden vorstehend genannten Patentanmeldungen der Anmelderin lässt sich ein TEDA von vorzüglicher Reinheit und Qualität erhalten. Der letzte Verfahrensschritt, also die Kristallisation des TEDA aus der nach dem Quench erhaltenen Lösung, ist in einigen Fällen unumgänglich, beispielsweise dann, wenn TEDA in sehr hoher Qualität und Reinheit gewünscht wird. Soll ein solches TEDA in fester Form vorliegen, ist dieser Kristallisationsschritt oder zumindest ein Isolieren des TEDA aus der Lösung ein obligatorischer Verfahrensschritt und nicht weiter störend. Jedoch wird TEDA häufig in Form einer Lösung in entsprechenden Anwendungen eingesetzt, insbesondere bei Verwendung als Katalysator bei der Polyurethan-Herstellung. Dazu muss das zuvor aus einer Lösung auskristallisierte TEDA wieder aufgelöst werden. Es ist offensichtlich, dass erwünscht ist, über ein Verfahren der Herstellung von hochreinem TEDA zu verfügen, das ein solches, hohen Anforderungen genügendes TEDA ohne den Kristallisationsschritt liefert.

Die Aufgabe der vorliegenden Erfindung besteht darin, ein Verfahren zur Verfügung zu stellen, das die Gewinnung von hochreinem TEDA bzw. von hochreinen TEDA-Lösungen gestattet, ohne dass nach dem TEDA-Quench eine Kristallisation notwendig ist.

Diese Aufgabe wird gelöst durch ein Verfahren zur Gewinnung von hochreinem TEDA, bei dem rohes TEDA von Schwersiedem befreit und anschließend das TEDA verdampft und in ein flüssiges Lösungsmittel eingeleitet wird.

Es wurde gefunden, dass ein TEDA hoher Reinheit erhalten wird, wenn vor dem Verdampfen und Einleiten des TEDA in ein Lösungsmittel (TEDA-Quench) die Schwersieder abgetrennt werden.

Vorzugsweise wird der Schritt des vor dem Einleiten in ein Lösungsmittel erfolgenden Verdampfens als Destillation durchgeführt, insbesondere als Reindestillatiori des TEDA. Bei dieser Destillation werden die Komponenten, die für eine Verschlechterung der Qualität des TEDA verantwortlich sind, nicht oder nur in untergeordneten Mengen gebildet, wenn erfindungsgemäß zuvor die Schwersieder abgetrennt wurden.

Bei den bisher bekannten Verfahren zur Gewinnung von reinem TEDA durch TEDA-Quench wurde das rohe TEDA verdampft, generell destilliert. Hierbei lagen die bei der vorherigen Synthese des TEDA entstandenen Schwersieder in dem Sumpf der Destillation vor. Das nach dem Quench erhaltene TEDA wies noch Verunreinigungen auf, die eine Kristallisation des TEDA aus der erhaltenen Lösung notwendig machten.

Mit dem erfindungsgemäßen Verfahren dagegen wird nach dem Quench eine Lösung erhalten, die ein TEDA hoher Reinheit enthält und die generell direkt eingesetzt werden kann, beispielsweise als Katalysator bei der Polyurethan-Herstellung. Natürlich kann aus der nach dem Quench erhaltenen Lösung das TEDA auch in Substanz nach Kristallisation isoliert werden. Das auf diese Weise zugängliche TEDA weist dann eine hohe Reinheit auf. Es werden derart Reinheitsgrade von > 90 %, vorzugsweise > 95 %, insbesondere > 99 %, erreicht.

Das Abtrennen der Schwersieder kann mit geeigneten, dem Fachmann bekannten Verfahren der Trennung hochsiedender und leichtsiedender Komponenten erfolgen. Vorzugsweise erfolgt die Trennung durch Abdestillieren der Leichtsieder einschließlich TEDA von der nach der TEDA-Synthese erhaltenen Lösung. Dabei verbleiben die Schwersieder im Sumpf. Die Schwersieder werden häufig verworfen, können aber gewünschtenfalls auch wiederaufgearbeitet werden.

Aus der Leichtsieder-Fraktion wird das TEDA anschließend durch Destillation abgetrennt. Bei dieser Destillation soll vorzugsweise eine Temperatur von < 200°C, insbesondere < 180°C, eingehalten werden. Bei diesen Temperaturen werden vorteilhaft geringe Mengen an Nebenprodukt gebildet. Werden die genannten Temperaturen wesentlich überschritten, so bilden sich im allgemeinen unerwünscht hohe Mengen an Nebenprodukt. Der Druck, der bei dieser Destillation herrscht, liegt generell bei Werten von 0,5 bis 1,5 bar. Nach dem Abdestillieren wird das dampfförmige TEDA, das erfindungsgemäß eine Reinheit > 90 Gew.-%, vorzugsweise > 95 Gew.%, insbesondere > 99 Gew.-% besitzt, in ein flüssiges Lösungsmittel eingeleitet. Bei diesem Quench wird erfindungsgemäß reines TEDA erhalten. Dieses kann, wie oben erwähnt, in Form der nach dem Quench erhaltenen Lösung eingesetzt oder gewünschtenfalls aus diesem durch Kristallisation in an sich bekannter Weise erhalten werden.

In einer Variante der vorliegenden Erfindung wird das nach dem Abtrennen der Schwersieder-Fraktion anfallende Gemisch nicht direkt aufgetrennt und das dabei erhaltene TEDA einem Quench unterworfen, sondern das von Schwersiedem befreite Gemisch wird zunächst gemäß der DE 101 00 943.7 in einem Lösungs- oder Verdünnungsmittel gelöst. Aus der so erhaltenen Lösung wird das TEDA dann abdestilliert und gequencht. Diese Variante wird vorzugsweise dann eingesetzt, wenn eine besonders reine TEDA-Qualität erwünscht ist.

In einer weiteren Variante der vorliegenden Erfindung werden zunächst die niedriger als TEDA siedenden Leichtsieder, beispielsweise Ammoniak, Ethylamin oder Wasser, durch Destillation von dem restlichen, nach Synthese erhaltenen Produktgemisch abgetrennt. Dabei werden vorzugsweise Temperaturen von 95 bis 120°C und Drücke von 0,5 bis 1,5 bar eingehalten. Von dem nach der Abtrennung der genannten Leichtsieder erhaltenen Gemisch werden dann die Schwersieder abgetrennt und die verbleibenden Leichtsieder enthaltend TEDA erfindungsgemäß aufgearbeitet. Dabei können auch die möglichen Verfahrensvarianten, beispielsweise das Verdampfen aus einem Lösungs- oder Verdünnungsmittel, eingesetzt werden.

Im übrigen wird das erfindungsgemäße Verfahren so durchgeführt, wie es in den Anmeldungen DE 199 33 850.7, 199 62 455.0 und DE 101 00 943.7 der Anmelderin beschrieben wird. Die in den genannten Anmeldungen beschriebenen Verfahrensschritte der Reinigung von TEDA, die nicht das Abtrennen der Schwersieder umfassen, sind ein integraler Bestandteil des Verfahrens nach der vorliegenden Anmeldung. Die Verfahren werden nachfolgend noch einmal kurz dargestellt.

Durch das Einleiten des dampfförmigen TEDA in ein flüssiges Lösungsmittel (TEDA-Quench) wird die Bildung von unerwünschten Nebenprodukten, die zur Qualitätsminderung führen, entscheidend verringert.

Als Lösungsmittel für diesen TEDA-Quench eignen sich eine Vielzahl organischer Lösungsmittel. Beispiele umfassen aliphatische, cyclische oder acyclische Kohlenwasserstoffe, insbesondere cyclische und acyclische, verzweigte oder unverzweigte Alkane oder Alkangemische, beispielsweise n-Pentan, i-Pentan, Cyclopentan, Hexan, Cyclohexan, Heptan, Octan und Petrolether, chlorierte aliphatische Kohlenwasserstoffe, insbesondere chlorierte Alkane, beispielsweise Dichlormethan, Trichlormethan, Dichlorethan und Trichlorethan, aromatische Kohlenwasserstoffe, beispielsweise Benzol, Toluol und Xylole, chlorierte aromatische Kohlenwasserstoffe, beispielweise Chlorbenzol, Alkohole, beispielsweise Methanol, Ethanol, Ethylenglykol, 1,4-Butandiol und Polyetheralkohole, insbesondere Polyalkylenglykole, beispielsweise Diethylenglykol und Dipropylenglykol, Ketone, insbesondere aliphatische Ketone, beispielsweise Aceton, Methylethylketon und Diethylketon, aliphatische Carbonsäureester, beispielsweise Essigsäuremethylester und Essigsäureethylester, aliphatische Nitrile, beispielsweise Acetonitril und Propionitril, Ether, beispielsweise Dioxan, THF, Diethylether und Ethylenglykoldimethylether sowie Gemische der vorstehend aufgeführten Lösungsmittel.

Als Lösungsmittel für den TEDA-Quench wird vorzugsweise ein aliphatischer Kohlenwasserstoff oder ein Polyalkylenglykol, insbesondere ein gesättigter cyclischer oder acyclischer, aliphatischer Kohlenwasserstoff mit 5 bis 8 C-Atomen, beispielsweise Pentan, Hexan, Cyclohexan oder Heptan, oder Dipropylenglykol, verwendet. Die optionale Kristallisation des reinen TEDA aus der erfindungsgemäß hergestellten TEDA-Lösung kann nach dem dem Fachmann bekannten Verfahren erfolgen. Die durch eine nachfolgende mehrstufige, vorzugsweise einstufige Kristallisation erhaltenen TEDA-Kristalle sind hochrein.

Das Einleiten des dampfförmigen TEDA in das flüssige Lösungsmittel erfolgt in einem Quench-Apparat, vorzugsweise einem Fallfilmkondensator (Dünnschicht-, Rieselfilm-oder Fallstromkondensator) oder in einem Düsenapparat. Dabei kann das dampfförmige TEDA im Gleich- oder im Gegenstrom mit dem flüssigen Lösungsmittel geführt werden. Vorteilhaft ist die Einleitung des dampfförmigen TEDA von oben in den Quenchapparat. Weiterhin vorteilhaft ist die tangentiale Zufuhr des flüssigen Lösungsmittels am Kopf des Fallfilmkondensators oder die Zufuhr des flüssigen Lösungsmittels durch eine oder mehrere Düsen, um eine vollständige Benetzung der Innenwand des Quenchapparates zu erreichen.

Die Menge des verwendeten Lösungsmittels wird nach Zweckmäßigkeitsgesichtspunkten ausgewählt. Im allgemeinen wird so verfahren, dass, je nach Art des Lösungsmittels, Lösungen mit einem TEDA-Gehalt von ca. 1 bis 50 Gew.-%, vorzugsweise 20 bis 40 Gew.-%, erhalten werden.

Im allgemeinen wird die Temperatur im TEDA-Quench durch Temperieren des eingesetzten Lösungsmittels und/oder des Quenchapparates auf 20 bis 100°C, vorzugsweise 30 bis 60°C, eingestellt.

Der Absolutdruck im TEDA-Quench beträgt im allgemeinen 0,5 bis 1,5 bar.

Wird bei der Reinigung des TEDA dieses entsprechend der DE 101 00 943.7 aus einer Mischung mit einem Lösungs- oder Verdünnungsmittel verdampft, weist das Lösungs-oder Verdünnungsmittel vorzugsweise bei Normaldruck einen Siedepunkt von 180 bis 250°C, mehr bevorzugt von 180 bis 230°C, insbesondere von 190 bis 210°C auf.

Als Lösungs- oder Verdünnungsmittel, das die Mischung enthält, aus der das TEDA verdampft wird, eignen sich besonders inerte
- polare aprotische Lösungsmittel beispielsweise Alkyl-2-pyrrolidone, beispielsweise N-Methyl-2-pyrrolidon (NMP), 1-Ethyl-2-pyrrolidon, 1,5-Dimethyl-2-pyrrolidon, 1-Isopropyl-2-pyrrolidon, Ether, beispielsweise Diethylenglykoldiethylether, Triethylenglykoldimethylether und Triethylenglykoldiethylether, Ketone, beispielsweise Acetophenon und Propiophenon, Lactone, beispielsweise γ-Butyrolacton, Sulfoxide, beispielsweise Dimethylsulfoxid, Carbonsäureester, beispielsweise Fumarsäuredimethylester, Nitrile, beispielsweise Benzonitril, und Harnstoffe, beispielsweise 1,3-Dimethyl-imidazolidin-2-on (DMEU) und Tetramethylharnstoff,
- cyclische oder acyclische Kohlenwasserstoffe, insbesondere gesättigte cyclische oder acyclische Kohlenwasserstoffe, beispielsweise Undecan, Dodecan, cis-Dekalin und trans-Decalin,
- chlorierte aliphatische Kohlenwasserstoffe beispielsweise 1-Chloroctan und 1,1-Dichloroctan,
- aromatische Kohlenwasserstoffe, Nitroaromaten und Phenole, beispielsweise Naphthalin, n-Butylbenzol, Phenol, Kresol, Nitrobenzol und Nitrophenol,
- chlorierte aromatische Kohlenwasserstoffe, beispielsweise 1,2-Dichlorbenzol, Benzylchlorid, 1,2,3,4-Tetramethylbenzol und 1,2,3,5-Tetramethylbenzol,
- Alkohole, beispielsweise Benzylalkohol, 2-Ethylhexanol, 1-Octanol, i-Decanol, 1,2-Propandiol, 1,3-Propandiol, Ethylenglykol, Diethylenglykol, 1,2-Propylenglykol, 1,3-Propylenglykol, 1,2-Butandiol, 1,3-Butandiol, 2,3-Butandiol, 1,4-Butandiol, Neopentylglykol, Diethylenglykolmonomethylether und Dipropylenglykol,
- primäre, sekundäre und tertiäre Amine, beispielsweise Tri-n-Butylamin, Benzylamin, Anilin, N-Ethylanilin, N,N-Dimethylanilin und N,N-Diethylanilin,
- N-Alkylamide, beispielsweise N-Methylformamid und N-Methylacetamid
und deren Gemische.

Besonders bevorzugt sind polare aprotische Lösungs- oder Verdünnungsmittel mit einem E^{N}_{T}-Wert von 0,1 bis 0,6, besonders von 0,2 bis 0,5, insbesondere von 0,3 bis 0,45.

(Zur Definition des E^{N}_{T}-Wertes siehe Ch. Reichardt, Solvents and solvent effects in organic chemistry, 2. Auflage, VCH 1988).

Ganz besonders bevorzugte Lösungsmittel sind NMP und Ethylenglykol.

Das Lösungs- oder Verdünnungsmittel, das die Mischung enthält, aus der das TEDA verdampft wird, wird bevorzugt nach dem Abtrennen der Schwersieder dem noch verunreinigten TEDA zugesetzt.

Das Lösungs- oder Verdünnungsmittel kann im einmaligen Durchlauf oder nach der Abtrennung der Schwersieder als Kreislauflösung eingesetzt werden.

Die Menge des verwendeten Lösungs- oder Verdünnungsmittel wird nach Zweckmäßigkeitsgesichtspunkten ausgewählt. Im allgemeinen wird so verfahren, dass je nach Art des Lösungs- oder Verdünnungsmittels Lösungen oder Mischungen mit einem TEDA-Gehalt von ca. 1 bis 90 Gew.-%, bevorzugt 40 bis 70 Gew.-%, erhalten werden.

Das Verdampfen des TEDA, optionsweise aus einer Mischung von diesem mit einem Lösungs- oder Verdünnungsmittel kann nach den dem Fachmann geläufigen Verfahren und Bedingungen erfolgen, z.B. in einer Destillations- oder Rektifikationsapparatur, wobei das TEDA gegebenenfalls zusammen mit dem Lösungs- oder Verdünnungsmittel vorgelegt wird.

Bevorzugt wird das dampfförmige TEDA am Kopf oder in einem Seitenabzug einer Destillationskolonne erhalten. Das dampfförmige TEDA im erfmdungsgemäßen Verfahren besitzt im allgemeinen eine Reinheit von größer 90 Gew.-%, bevorzugt größer 95 Gew.-%, insbesondere größer 99 Gew.-%.

Die Zeitdauer zwischen Anfall des im erfindungsgemäßen Verfahren verwendeten dampfförmigen TEDA und TEDA-Quench beträgt vorteilhafterweise ≤ 10 Sekunden. Das zu reinigende TEDA kann nach bekannten Verfahren, z.B. durch Umsetzung von Monoethanolamin, Diethanolamin, Triethanolamin, Ethylendiamin, Diethylentriamin, Triethylentetramin, Piperazin, N-(2-Hydroxyethyl)-piperazin, N,N'-Bis(2-Hydroxyethyl)-piperazin, N-(2-Aminoethyl)-piperazin, N,N'-Bis(2-Aminoethyl)-piperazin, Morpholin oder Mischungen hiervon an einem Katalysator, beispielsweise Metallpyrophosphate, Metallphosphate, beispielsweise Erdalkalimonohydrogenphosphat, Zeolithe, Zirkoniumphosphate, Al₂O₃, SiO₂, phosphorhaltiges TiO₂ oder ZrO₂ bei erhöhter Temperatur, im allgemeinen 250 bis 450°C, erhalten werden. Üblicherweise beträgt hierbei der Druck 0,1 bis 50, insbesondere 0,1 bis 5 bar. Optional kann die Umsetzung in Gegenwart eines inerten polaren aprotischen Lösungsmittels, wie N-Allcylpyrrolidonen, beispielsweise N-Methylpyrrolidon, Dioxan, THF, Dialkylfbrmamiden, beispielsweise Dimethylformamid, Dialkylacetamiden, beispielsweise Dimethylacetamid und eines inerten Trägergases beispielsweise N₂ oder Ar durchgeführt werden.

Optionsweise kann das TEDA auskristallisiert und durch Fest-Flüssig-Trennung abgetrennt werden.

Gemäß einer bevorzugten Ausführungsform lässt sich das erfindungsgemäße Verfahren wie folgt ausführen:

Eine Mischung enthaltend TEDA, die beispielsweise als Reaktionsaustrag in einem kontinuierlichen Verfahren durch Umsetzung von Ethylendiamin und Piperazin in einem Gasphasenreaktor bei 320 bis 420°C und 0,5 bis 1,5 bar in Gegenwart eines Lösungsmittels, beispielsweise Wasser, eines Trägergases, beispielsweise N₂ oder Ar und eines Zeolithkatalysators erhalten werden kann, beispielsweise gemäß der Patentanmeldung DE 100 61 863.4, wird in eine Destillationsapparatur mit einer Destillationskolonne mit zum Beispiel ca. 15 theoretischen Stufen geleitet. Hier werden Leichtsieder, beispielsweise Ammoniak, Ethylamin oder Wasser bei einer Kopftemperatur von 95 bis 120°C und einem Druck von 500 mbar bis 1,5 bar über Kopf abgetrennt. Der Sumpfablauf wird in eine weitere Destillationskolonne mit ca. 30 theoretischen Stufen gepumpt. Bei einem Druck von 500 mbar bis 1,5 bar werden in dieser Kolonne die Schwersieder über den Sumpfablauf ausgeschleust. Bei einer Kopftemperatur von 150 bis 170°C werden TEDA und Piperazin in eine weitere Destillationskolonne mit ca. 30 theoretischen Stufen gepumpt. Bei einem Druck von 500 mbar bis 1,5 bar wird in dieser Kolonne Piperazin über den Kopfabzug abgetrennt und optional wieder zurück zum Synthesereaktor gefahren.

Der Sumpfablauf, der Spuren an unerwünschten Zersetzungsprodukten enthält, wird teilweise ausgeschleust und/oder zur vorhergehenden Kolonne zurückgeführt.

In einem Seitenabzug der Kolonne wird TEDA mit einer Reinheit von > 95 Gew.-%, insbesondere > 99 Gew.-%, über einen Teilkondensator dampfförmig abgezogen und in einem Fallfilmkondensator in einem Lösungsmittel, beispielsweise Dipropylenglykol, 1,4-Butandiol oder Monoethylenglykol, bei einer Temperatur von 30 bis 100°C, bevorzugt 30 bis 60°C, direkt schockartig abgekühlt und gleichzeitig gelöst (TEDA-Quench).

### Beispiele:

### Beispiel 1 (Vergleichsbeispiel, nicht erfindungsgemäß)

Die Versuche wurden in einen mit elektrischen Heizbändern beheizten 4 1 (Katalysatorvolumen) Salzbadreaktor aus rostfreiem Stahl durchgeführt. Als Katalysator wurde ein Zeolith in Form von Strängen (Durchmesser ca. 2 mm, Länge ca. 30 mm) verwendet (Katalysatorschüttung).

Das Einsatzgut von 1000g/h sowie 3 Nl/h (Nl = Normliter = auf Normalbedingungen umgerechnetes Volumen) Stickstoff wurden bei Normaldruck in den auf 350°C beheizten Salzbadreaktor geleitet (Katalysatorbelastung: 0,3 kg Einsatzgut pro 1 Kat. (Schüttvolumen) und pro h).

Das Einsatzgut hatte folgende Zusammensetzung (Angaben in Gew.-%):

| | |
|---|---|
| Ethylendiamin | 30% |
| Piperazin | 20% |
| Wasser | 50% |

Das dampfförmige Reaktionsprodukt wurde in einem Quench mit Kreislaufflüssigkeit, die aus zuvor erhaltenem flüssigem Reaktionsprodukt bestand (siehe unten), bei 80°C kondensiert.

Die Analyse des Kondensats ergab folgende Zusammensetzung (Angaben in Gew.-%):

| | |
|---|---|
| Ammoniak | 3% |
| Piperazin | 17% |
| Triethylendiamin | 23% |
| Wasser | 54% |
| Rest | Schwersieder und andere Nebenprodukte |

Die nicht kondensierten Anteile wurden nach einem Gas-Flüssig-Abscheider in eine Destillationskolonne (K200) abgeleitet.

Ein Teil des flüssigen Reaktionsproduktes wurde gekühlt und als Flüssigkeitskreislauf (für den Reaktionsaustrags-Quench) verwendet. Ein anderer Teil wurde kontinuierlich mittels einer Pumpe in eine weitere Destillationskolonne K200 gepumpt. Die Glaskolonne mit einem Durchmesser von 50 mm war mit 30 Glockenböden ausgerüstet. Das Rücklaufverhältnis betrug etwa 1:1.

Die Leichtsieder (Ammoniak, Ethylamin, Wasser) wurden bei Normaldruck und einer Kopftemperatur von 96°C am Kopf der Kolonne flüssig abgezogen.

Der Sumpfablauf der Destillationskolonne wurde bei 155°C kontinuierlich in eine nachfolgende Destillationskolonne (K300) gepumpt.

Die Glaskolonne K300 mit einem Durchmesser von 50 mm war mit 60 Glockenböden ausgerüstet. Das Rücklaufverhältnis betrug etwa 10:1. Piperazin wurde bei Normaldruck und einer Kopftemperatur von 150°C am Kopf der Kolonne flüssig abgezogen und zum Reaktor zurückgefahren.

Der Sumpfablauf der Destillationskolonne K300 wurde bei 184°C kontinuierlich in eine weitere Destillationskolonne (K400) gepumpt.

Die Analyse des Sumpfablaufs ergab folgende Zusammensetzung (Angaben in Gew.-%):

| | |
|---|---|
| Piperazin | 0,2% |
| Triethylendiamin (TEDA) | 83% |
| Rest | Schwersieder und andere Nebenprodukte |

Die Glaskolonne K400 mit einem Durchmesser von 50 mm war mit 50 Glockenböden ausgerüstet. Das Rücklaufverhältnis betrugt etwa 8:1.

Die Schwersieder wurden kontinuierlich bei 230°C über den Kolonnensumpf ausgeschleust, die Vorlauftemperatur des ölbeheizten Verdampfers betrug 260°C. Am Kopf der Kolonne wurde TEDA dampfförmig abgezogen und bei ca. 30°C im Lösungsmittel Dipropylenglykol schockartig abgekühlt und gleichzeitig gelöst (TEDA-Quench). Für den TEDA-Quench wurde ein Fallfilmkondensator (Rieselfilm oder Fallstromkondensator) eingesetzt, bei dem dampfförmiges TEDA von oben eingeleitet wurde. Die Dipropylenglykolzufuhr erfolgte tangential am Kopf des Fallfilmkondensators. Die resultierende Lösung hatte folgende Zusammensetzung (Angaben in Gew.-%):

| | |
|---|---|
| Piperazin | 0,7% |
| Ethylpiperazin | 0,08% |
| Triethylendiamin (TEDA) | 30,0% |
| Dipropylenglykol | 68,5% |
| Rest | Nebenprodukte |

Das so gewonnene TEDA wies bezüglich seiner Farbe und seines Geruches ungenügende Eigenschaften auf und war deshalb nicht marktfähig.

Diese TEDA/DPG-Lösung besaß eine APHA-Farbzahl von 80.

Das erhaltene TEDA wies einen Geruch von cyclischen gesättigten 5-Ring-N-Heterocyclen oder anderen cyclischen 6-Ring-N-Heterocyclen und/oder aromatischen 5-oder 6-Ring-N-Heterocyclen auf.

Die erforderliche hohe Temperatur im Abtriebsteil der letzten Destillationskolonne (Produkttemperatur bis zu 230°C) führte zu erheblichen thermischen Belastungen des TEDA und der Schwersieder und dadurch zur Bildung unerwünschter Zersetzungsprodukte. Durch Bilanzierung der Zulauf- und Ablaufströme der Kolonne kann auf eine Piperazin-Quelle im Sumpf der Kolonne K400 geschlossen werden. Als PIP-Quelle im Sumpf der K400 wird die Zersetzung von Hochsiedern (z.B. Aminoethylpiperazin, 1,2-Dipiperazinethan) angenommen.

### Beispiel 2 (erfindungsgemäß):

Bei Durchführung des Versuchs wie in Beispiel 1 beschrieben, jedoch wurden von dem Sumpfablauf der Destillationskolonne K200 zunächst in der nachfolgenden Destillationskolonne K300 die Schwersieder abgetrennt.

Die Glaskolonne K300 mit einem Durchmesser von 50 mm war mit 60 Glockenböden ausgerüstet. Das Rücklaufverhältnis betrug etwa 6:1. Die Schwersieder wurden kontinuierlich bei 220°C über den Kolonnensumpf ausgeschleust, die Vorlauftemperatur des ölbeheizten Verdampfers betrug 240°C.

Die Analyse des Sumpfablaufs ergab folgende Zusammensetzung (Angaben in Gew.-%):

| | |
|---|---|
| Piperazin | 63% |
| Triethylendiamin | 36% |
| Rest | Nebenprodukte |

Die Glaskolonne K400 mit einem Durchmesser von 50 mm war mit 60 Glockenböden ausgerüstet. Das Rücklaufverhältnis betrug etwa 8:1. Am Kopf der Kolonne wurde Piperazin kontinuierlich bei 148°C ausgeschleust und zum Reaktor zurückgefahren. TEDA wurde dampfförmig aus dem Seitenabzug abgezogen und bei ca. 30°C im Lösungsmittel Dipropylenglykol schockartig abgekühlt und gleichzeitig gelöst (=TEDA-Quench). Für den TEDA-Quench wurde ein Fallfilmkondensator (Rieselfilm- oder Fallstromkondensator) eingesetzt, bei dem dampfförmiges TEDA von oben eingeleitet wurde.

Dipropylenglykol wurde am Kopf des Fallfilmkondensators eingedüst. Die resultierende Lösung hatte folgende Zusammensetzung (Angaben in Gew.-%):

| | |
|---|---|
| Piperazin | 0,01 % |
| Ethylpiperazin | 0,01% |
| Triethylendiamin (TEDA) | 34,0% |
| Dipropylenglykol | 65,9% |
| Rest | Nebenprodukte |

Diese TEDA/DPG-Lösung besaß eine APHA-Farbzahl von 32 und kann direkt als Katalysator bei der Herstellung von Polyurethanen eingesetzt werden.

Das erhaltene TEDA wies keinen Geruch nach cyclischen gesättigten 6-Ring-N-Heterocyclen und/oder aromatischen 5- oder 6-Ring-N-Heterocyclen auf. Eine weitere Aufarbeitung durch eine nachfolgende Kristallisation ist nicht notwendig, kann jedoch gewünschtenfalls durchgeführt werden, wobei ein hochreines TEDA erhalten wird.

## Patentansprüche

1. Verfahren zur Gewinnung von Triethylendiamin, das einen Reinheitsgrad von > 90 % aufweist, **dadurch gekennzeichnet, dass** rohes Triethylendiamin von Schwersiedem befreit und anschließend das Triethylendiamin aus dem derart erhaltenen Gemisch verdampft und in ein flüssiges Lösungsmittel eingeleitet wird.

2. Verfahren nach Anspruch 1, **dadurch gekennzeichnet, dass** das Verdampfen des Triethylendiamins als Destillation durchgeführt wird, insbesondere als Reindestillation.

3. Verfahren nach Anspruch 1 oder 2, **dadurch gekennzeichnet, dass** die Trennung der Schwersieder von den Leichtsiedern durch Abdestillieren der Leichtsieder einschliesslich Triethylendiamin von der nach der Triethylendiamin-Synthese erhaltenen Lösung erfolgt, wobei die Schwersieder im Sumpf verbleiben.

4. Verfahren nach einem der Ansprüche 1 bis 3, **dadurch gekennzeichnet, dass** vor der Abtrennung der Schwersieder die niedriger als Triethylendiamin siedenden Leichtsieder, vorzugsweise Ammoniak, Ethylamin und/oder Wasser, durch Destillation von dem restlichen, nach Synthese erhaltenen Produktgemisch abgetrennt werden.

5. Verfahren nach einem der Ansprüche 1 bis 4, **dadurch gekennzeichnet, dass** nach dem Abtrennen der Schwersieder das Triethylendiamin bei einer Temperatur von <200 °C, vorzugsweise <180 °C, und einem Druck von 0,5 bis 1,5 bar abdestilliert wird.

6. Verfahren nach einem der Ansprüche 1 bis 5, **dadurch gekennzeichnet, dass** das nach dem Abtrennen der Schwersieder-Fraktion anfallende Gemisch zunächst in einem Lösungs- oder Verdünnungsmittel gelöst und aus der so erhaltenen Lösung das Triethylendiamin dann abdestilliert und in ein flüssiges Lösungsmittel geleitet wird.

7. Verfahren nach einem der Ansprüche 1 bis 6, **dadurch gekennzeichnet, dass** als Lösungsmittel für den Triethylendiamin-Quench ein Lösungsmittel aus der Gruppe aliphatische, cyclische und acyclische Kohlenwasserstoffe, insbesondere cyclische und acyclische, verzweigte und unverzweigte Alkane und Alkangemische, chlorierte aliphatische Kohlenwasserstoffe, insbesondere chlorierte Alkane, aromatische Kohlenwasserstoffe, insbesondere Benzol, Toluol und Xylole, chlorierte aromatische Kohlenwasserstoffe, insbesondere Chlorbenzol, Alkohole, vorzugsweise Methanol, Ethanol, Ethylenglykol, 1,4-Butandiol und Polyetheralkohole, insbesondere Polyalkylenglykole, Ketone, vorzugsweise aliphatische Ketone, insbesondere Aceton, Methylethylketon und Diethylketon, aliphatische Carbonsäureester, vorzugsweise Essigsäuremethylester und Essigsäureethylester, aliphatische Nitrile, vorzugsweise Acetonitril und Propionitril, Ether, vorzugsweise Dioxan, THF, Diethylether und Ethylenglykoldimethylether sowie Gemischen der vorstehend aufgeführten Lösungsmittel eingesetzt wird.

8. Verfahren nach einem der Ansprüche 1 bis 7, **dadurch gekennzeichnet, dass** das Einleiten des dampfförmigen Triethylendiamin in das flüssige Lösungsmittel in einem Quench-Apparat, vorzugsweise einem Fallfilmkondensator oder in einem Düsenapparat, erfolgt, wobei das dampfförmige Triethylendiamin im Gleich- oder im Gegenstrom mit dem flüssigen Lösungsmittel geführt wird, vorzugsweise dieses von oben in den Quenchapparat geleitet wird.

9. Verfahren nach einem der Ansprüche 1 bis 8, **dadurch gekennzeichnet, dass** das Lösungs- oder Verdünnungsmittel, das die Mischung enthält, aus der das Triethylendiamin verdampft wird, ausgewählt ist aus der Gruppe bestehend aus polaren aprotische Lösungsmitteln, Ethern, Ketonen, Lactonen, Sulfoxiden, Carbonsäureestern, Nitrilen, Harnstoffen, cyclische und acyclischen Kohlenwasserstoffen, insbesondere gesättigten cyclischen oder acyclischen Kohlenwasserstoffe, chlorierten aliphatischen Kohlenwasserstoffen, aromatischen Kohlenwasserstoffen, Nitroaromaten und Phenolen, chlorierten aromatischen Kohlenwasserstoffen, Alkoholen, primären, sekundären und tertiären Aminen, N-Alkylamiden, und deren Gemischen, insbesondere polaren aprotischen Lösungs- oder Verdünnungsmitteln mit einem E^{N}_{T}_ Wert von 0,1 bis 0,6, besonders von 0,2 bis 0,5, insbesondere von 0,3 bis 0,45.

## Claims

1. A process for obtaining triethylenediamine having a purity of > 90%, wherein crude triethylenediamine is freed from high boilers and then the triethylenediamine is vaporized from the mixture thus obtained and is passed into a liquid solvent.

2. The process according to claim 1, wherein the vaporization of the triethylenediamine is carried out in the form of a distillation, in particular a distillation for purification.

3. The process according to claim 1 or 2, wherein the separation of the high boilers from the low boilers is effected by distilling off the low boilers, including triethylenediamine, from the solution obtained after the triethylenediamine synthesis, the high boilers remaining in the bottom product.

4. The process according to any of claims 1 to 3, wherein, before the high boilers are separated off, the low boilers having a boiling point lower than that of triethylenediamine, preferably ammonia, ethylamine and/or water, are separated by distillation from the remaining product mixture obtained after synthesis.

5. The process according to claims 1 to 4, wherein the triethylenediamine is distilled off at < 200°C, preferably < 180°C, and from 0.5 to 1.5 bar after the high boilers have been separated off.

6. The process according to any of claims 1 to 5, wherein the mixture obtained after the high boiler fraction has been separated off is first dissolved in a solvent or diluent and the triethylenediamine is then distilled off from the solution thus obtained and is passed into a liquid solvent.

7. The process according to any of claims 1 to 6, wherein a solvent from the group consisting of aliphatic, cyclic and acyclic hydrocarbons, in particular cyclic and acyclic, branched and straight-chain alkanes and alkane mixtures, chlorinated aliphatic hydrocarbons, in particular chlorinated alkanes, aromatic hydrocarbons, in particular benzene, toluene and xylenes, chlorinated aromatic hydrocarbons, in particular chlorobenzene, alcohols, preferably methanol, ethanol, ethylene glycol and 1,4-butanediol, and polyether alcohols, in particular polyalkylene glycols, ketones, preferably aliphatic ketones, in particular acetone, methyl ethyl ketone and diethyl ketone, aliphatic carboxylic esters, preferably methyl acetate and ethyl acetate, aliphatic nitriles, preferably acetonitrile and propionitrile, ethers, preferably dioxane, tetrahydrofuran, diethyl ether and ethylene glycol dimethyl ether, and mixtures of the abovementioned solvents are used as solvents for the triethylenediamine quench.

8. The process according to any of claims 1 to 7, wherein the triethylenediamine vapor is passed into the liquid solvent in a quench apparatus, preferably a falling-film condenser, or in a nozzle apparatus, the triethylenediamine vapor being fed cocurrently with or countercurrently to the liquid solvent, preferably said triethylenediamine vapor being passed into the quench apparatus from above.

9. The process according to any of claims 1 to 8, wherein the solvent or diluent which contains the mixture from which the triethylenediamine is vaporized is selected from the group consisting of polar aprotic solvents, ethers, ketones, lactones, sulfoxides, carboxylic esters, nitriles, ureas, cyclic and acyclic hydrocarbons, in particular saturated cyclic or acyclic hydrocarbons, chlorinated aliphatic hydrocarbons, aromatic hydrocarbons, nitroaromatics and phenols, chlorinated aromatic hydrocarbons, alcohols, primary, secondary and tertiary amines, N-alkylamides and mixtures thereof, in particular polar aprotic solvents or diluents having an E^{N}_{T} value of from 0.1 to 0.6, in particular from 0.2 to 0.5, especially from 0.3 to 0.45.

## Revendications

1. Procédé d'obtention de triéthylènediamine présentant un degré de pureté > 90%, **caractérisé en ce que** la triéthylènediamine brute est libérée des produits à haut point d'ébullition, puis la triéthylènediamine est évaporée du mélange ainsi obtenu et est conduite dans un solvant liquide.

2. Procédé selon la revendication 1, **caractérisé en ce que** l'évaporation de la triéthylènediamine s'effectue par distillation, en particulier par distillation pure.

3. Procédé selon la revendication 1 ou 2, **caractérisé en ce que** la séparation des produits à haut point d'ébullition des produits à bas point d'ébullition s'exécute par séparation par distillation des produits à bas point d'ébullition y compris la triéthylènediamine de la solution obtenue après la synthèse de la triéthylènediamine, les produits à haut point d'ébullition restant dans le bas de colonne.

4. Procédé selon l'une quelconque des revendications 1 à 3, **caractérisé en ce qu'**avant la séparation des produits à haut point d'ébullition, les produits à bas point d'ébullition entrant en ébullition à une température plus basse que la triéthylènediamine, de préférence l'ammoniac, l'éthylamine et/ou l'eau, sont séparés par distillation du mélange de produits restant obtenu après la synthèse.

5. Procédé selon l'une quelconque des revendications 1 à 4, **caractérisé en ce qu'**après la séparation des produits à haut point d'ébullition, la triéthylènediamine est séparée par distillation à une température < 200°C, de préférence < 180°C, et à une pression de 0,5 à 1,5 bar.

6. Procédé selon l'une quelconque des revendications 1 à 5, **caractérisé en ce que** le mélange obtenu après la séparation de la fraction des produits à haut point d'ébullition est d'abord dissous dans un solvant ou diluant et, de la solution ainsi obtenue, la triéthylènediamine est ensuite séparée par distillation et conduite dans un solvant liquide.

7. Procédé selon l'une quelconque des revendications 1 à 6, **caractérisé en ce qu'**on utilise comme solvant pour le refroidissement rapide de la triéthylènediamine un solvant choisi parmi le groupe des hydrocarbures aliphatiques, cycliques et acycliques, en particulier les alcanes et les mélanges d'alcanes cycliques et acycliques, ramifiés et non ramifiés, les hydrocarbures aliphatiques chlorés, en particulier les alcanes chlorés, les hydrocarbures aromatiques, en particulier le benzène, le toluène et les xylols, les hydrocarbures chlorés aromatiques, en particulier le chlorobenzène, les alcools, de préférence le méthanol, l'éthanol, l'éthylèneglycol, le 1,4-butanediol et les polyétheralcools, en particulier les polyalkylène-glycols, les cétones, de préférence les cétones aliphatiques, en particulier l'acétone, la méthyléthylcétone et la diéthylcétone, les esters d'acide carboxylique aliphatiques, de préférence l'acétate de méthyle et l'acétate d'éthyle, les nitriles aliphatiques, de préférence l'acétonitrile et le propionitrile, les éthers, de préférence le dioxane, le THF, le diéthyléther et l'éthylèneglycoldiméthyléther ainsi que des mélanges des solvants repris ci-dessus.

8. Procédé selon l'une quelconque des revendications 1 à 7, **caractérisé en ce que** la conduite de la triéthylènediamine sous forme de vapeur dans le solvant liquide s'effectue dans un appareil de refroidissement rapide, de préférence un condensateur à film tombant ou dans un appareil à buses, la triéthylènediamine sous forme de vapeur étant conduite à co-courant ou à contre-courant avec le solvant liquide, de préférence celle-ci sera conduite par le haut dans l'appareil de refroidissement rapide.

9. Procédé selon l'une quelconque des revendications 1 à 8, **caractérisé en ce que** le solvant ou le diluant, qui contient le mélange d'où la triéthylènediamine s'évapore, est choisi dans le groupe composé de solvants polaires aprotiques, des éthers, des cétones, des lactones, des sulfoxydes, des esters d'acide carboxylique, des nitriles, des urées, des hydrocarbures cycliques et acycliques, en particulier des hydrocarbures saturés cycliques ou acycliques, des hydrocarbures chlorés aliphatiques, des hydrocarbures aromatiques, des nitroaromatiques et des phénols, des hydrocarbures chlorés aromatiques, des alcools, des amines primaires, secondaires et tertiaires, des N-alkylamides et de leurs mélanges, en particulier des solvants ou diluants polaires aprotiques avec un indice E^{N}_{T}- de 0,1 à 0,6, particulièrement de 0,2 à 0,5, en particulier de 0,3 à 0,45.
